(19) Europäisches Patentamt
European Patent Office
Office européen des brevets

(11) **EP 4 679 446 A1**

(12) **EUROPEAN PATENT APPLICATION**

(43) Date of publication:
**14.01.2026 Bulletin 2026/03**

(21) Application number: **25166712.7**

(22) Date of filing: **27.03.2025**

(51) International Patent Classification (IPC):
**G16H 30/40** (2018.01)    **G16H 50/20** (2018.01)
**G16H 50/30** (2018.01)    **G16H 50/70** (2018.01)

(52) Cooperative Patent Classification (CPC):
**G16H 30/40; G16H 50/20; G16H 50/30;
G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **08.07.2024 US 202463668362 P**

(71) Applicant: **Siemens Healthineers AG
91301 Forchheim (DE)**

(72) Inventors:
• **PASSERINI, Tiziano
Plainsboro, 08536 (US)**
• **SHARMA, Puneet
Princeton Junction, 08550 (US)**

(74) Representative: **Kraus & Lederer PartGmbB
Thomas-Wimmer-Ring 15
80539 München (DE)**

(54) **FOUNDATION MODEL FOR PROCESSING PHYSIOLOGICAL MEASUREMENT DATA**

(57)    The invention relates to performing a training of a foundation model for processing measurement data of at least one physiological parameter associated with an anatomical target region. A method (800) comprises obtaining (802) text data (304, 404) describing one or more medical conditions associated with the anatomical target region; generating (804), using one or more physiological models associated with the anatomical target region, synthesized measurement data (308, 310, 402) of the at least one physiological parameter (302) based on the text data (304, 404); and training (808) the founda- tion model based on the text data (304, 404) and the synthesized measurement data (308, 310).

FIG 4

EP 4 679 446 A1

## Description

The present application relates to the field of a foundation model for processing measurement data of physiological parameters associated with an anatomical target region. In more detail, the present application relates to techniques for training of the foundation model, for example in the field of cardiac physiology measurement signals, and applying the trained foundation model for interpreting these measurement signals.

## BACKGROUND

Independent of the grammatical term usage, individuals with male, female or other gender identities are included within the term.

The analysis of temporal measurements of physiological phenomena is a pivotal aspect of cardiology, particularly when it comes to the non-invasive assessment of valve diseases such as stenosis and regurgitation. Diagnosing these conditions may rely heavily on the precise measurement and interpretation of multiple signals, each corresponding to different components of heart function. These signals may include, but are not limited to, blood flow velocities, transvalvular pressure gradients, time-varying valve opening diameter or area, which can be assessed using techniques such as echocardiography and magnetic resonance imaging.

However, the interpretation of these signals is not straightforward, as they often contain noise and artifacts that can significantly complicate their analysis. The challenge for healthcare professionals and technologists is not only to clean and extract relevant data from these signals but also to provide deeper insights into the underlying physiological processes. This involves understanding the significance of each signal in the context of the overall clinical picture, which may include patient history, symptoms, and other diagnostic findings.

For example, in the case of aortic valve stenosis, the measurement of aortic valve area and the velocity of blood flow through the valve may be deemed critical. These measurements may be interpreted in light of the patient's clinical status and the presence of any comorbid conditions. Similarly, for mitral regurgitation, the volume of blood flowing back into the left atrium and the resultant changes in atrial and ventricular size and function may be key indicators of disease severity and help guide treatment decisions.

Historically, the focus in cardiac signal analysis has been predominantly on improving the quality of signal acquisition and applying standard denoising techniques to ensure the clarity of the data collected. Denoising may include techniques for removing or reducing noise in the acquired signal and/or during signal acquisition. Despite these advancements in signal acquisition and denoising, there has not been a significant breakthrough in the interpretation of these signals that emphasizes their physiological significance within the context of the entire set of available measurements.

There is therefore a need to remedy the problems and shortcomings described above.

## SUMMARY

A computer-implemented method for performing a training of a foundation model for processing measurement data of at least one physiological parameter associated with an anatomical target region is provided. The method comprises obtaining text data describing one or more medical conditions associated with the anatomical target region, and generating, using one or more physiological models associated with the anatomical target region, synthesized measurement data of the at least one physiological parameter based on the text data. The foundation model is trained based on the text data and the synthesized measurement data.

A computer program comprises instructions which, when the program is executed by a training system, cause the training system to carry out the above computer-implemented method for performing a training of a foundation model.

A computer-readable medium comprises instructions which, when executed by a training system, cause the training system to carry out the above computer-implemented method for performing a training of a foundation model.

According to various examples, the foundation model comprises a first encoder and a second encoder. The training of the foundation model comprises determining, using the first encoder, one or more text embeddings associated with the text data, and determining, using the second encoder, one or more measurement embeddings associated with the synthesized measurement data. Parameter values of the foundation model are updated based on an association between the one or more text embeddings and the one or more measurement embeddings.

A training system for performing a training of a foundation model for processing measurement data of at least one physiological parameter associated with an anatomical target region comprises a training interface configured for obtaining text data describing one or more medical conditions associated with the anatomical target region. In addition, the training system comprises a training computation unit configured to generate, using one or more physiological models associated with the anatomical target region, synthesized measurement data of the at least one physiological parameter based on the text data, and to train the foundation model based on the text data and the synthesized measurement data. As an example, the text data may be obtained from the one or more physiological models.

Further, a computer-implemented method for establishing a diagnosis of a disease associated with a heart valve is provided. The method comprises obtaining a series of medical images depicting the heart valve, and determining, based on the series of medical images, measurement data of at least one physiological parameter associated with the heart valve. Further, according to the method, measurement embeddings associated with the measurement data are determined using the second encoder of the foundation model. The diagnosis of the disease is established based on the measurement embeddings, using a machine-learning model.

In various examples, the diagnosis of the disease may be established based on text embeddings that correspond to the measurement embeddings, e.g. using a machine-learning model.

A computer program comprises instructions which, when the computer program is executed by a providing system, cause the providing system to carry out the above computer-implemented method for establishing a diagnosis.

A computer-readable medium comprises instructions which, when executed by a providing system, cause the providing system to carry out the above computer-implemented method for establishing a diagnosis.

A providing system for establishing a diagnosis of a disease associated with a heart valve comprises an interface configured for obtaining a series of medical images depicting the heart valve and a computation unit. The computation unit is configured to determine, based on the series of medical images, measurement data of at least one physiological parameter associated with the heart valve. The computation unit is further configured to determine, using the second encoder of the above foundation model, measurement embeddings associated with the measurement data, and to establish, using a machine-learning model, the diagnosis of the disease based on the measurement embeddings.

The above summary is intended to give a brief overview of some possible implementations and should not be construed as limiting, as other implementations may include features other than those explicitly listed above.

BRIEF DESCRIPTION OF THE DRAWINGS

FIG. 1 schematically illustrates an artificial neural network according to various examples.

FIG. 2 schematically shows a convolutional neural network according to various examples.

FIG. 3 schematically illustrates generation of training samples according to various examples.

FIG. 4 schematically illustrates techniques used for multimodal contrastive learning according to various examples.

FIG. 5 schematically shows techniques for multimodal contrastive learning including an additional loss term according to various examples.

FIG. 6 schematically illustrates echocardiography images that may be processed for extracting signals applied to a trained foundation model for establishing a diagnosis according to various examples.

FIG. 7 schematically shows a training system according to various examples.

FIG. 8 shows a flowchart of a method for performing a training of a foundation model according to various examples.

FIG. 9 schematically shows a providing system according to various examples.

FIG. 10 shows a flowchart of a method for establishing a diagnosis according to various examples.

DETAILED DESCRIPTION

In the following, techniques are described with respect to the claimed methods as well as with respect to the claimed training and providing systems. Features, advantages or alternative embodiments herein can be assigned to the other claimed objects and vice versa. In other words, claims for the training and providing systems can be improved with features described or claimed in the context of the methods. In this case, the functional features of the method are embodied by objective units of the providing system.

Furthermore, in the following, techniques are described with respect to methods and systems performing a training of a foundation model as well as with respect to methods and systems that use the trained foundation model. Features, advantages or alternative embodiments herein can be assigned to the other claimed objects and vice versa. In other words, claims for methods and systems for training of the foundation model can be improved with features described or claimed in context of the methods and systems using the trained foundation model, and vice versa.

In general, a trained function mimics cognitive functions that humans associate with other human minds. In particular, by training based on training data the trained function is able to adapt to new circumstances and to detect and extrapolate patterns.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training. In particular, a trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

Various embodiments will be described in detail referring to the attached drawings. It should be noted that these embodiments serve as illustrative examples only and are not to be construed as limiting. For example, while embodiments may be described as comprising a plurality of features or elements, in other embodiments some of these features or elements may be omitted and/or may be replaced by alternative features or elements. Additionally, apart from the features or elements explicitly shown and described, further elements or features may be provided.

In the embodiments shown and described, any direct connections or couplings between elements or components, i.e. connections or couplings without intervening elements, may also be implemented by indirect connections or couplings, i.e. connections or couplings comprising one or more additional intervening elements, as long as the general function of the connection or coupling, for example to provide a certain kind of signal, a certain kind of information or to provide a certain kind of control, is essentially maintained. In other words, connections or couplings may be modified as long as the function or purpose of the connection or coupling remains essentially the same.

Features from different embodiments may be combined to form further embodiments. Variations, modifications and details described with respect to one of the embodiments may also be applied to other embodiments.

An aspect of the present disclosure relates to a computer-implemented method for training a foundation model designed to process measurement data of at least one physiological parameter associated with an anatomical target region. The term "foundation model" may refer to a pre-trained artificial intelligence model designed to process both text data (e.g., clinical notes or medical descriptions) and measurement data (e.g., physiological parameters like heart rate or oxygen saturation). The method involves obtaining text data that describes various medical conditions linked to the anatomical target region, such as heart valve functionalities or diseases like mitral regurgitation. The method then generates synthesized measurement data using one or more physiological models specific to the target region, ensuring these simulations reflect real-world conditions. This may optionally also include noise and artifacts. Finally, the foundation model is trained using the text data and the synthesized measurement data, enabling it to interpret complex signals effectively.

As an example, the text data may be obtained from the one or more physiological models.

This may enhance the interpretation of temporal measurements by capturing a wide array of physiological conditions, thereby improving diagnostic accuracy in cardiology. The use of physiological models allows for realistic simulations that include various pathologies and optionally noise, making the training dataset robust and comprehensive. Additionally, the multimodal approach combines signals with descriptive text, fostering a deeper understanding of the underlying physiology. This framework is particularly beneficial for assessing conditions like mitral regurgitation, where multiple measurements may be crucial for accurate diagnosis.

Furthermore, the model may be fine-tuned for specific tasks such as disease classification while maintaining a broad applicability across different physiological parameters. By leveraging extensive datasets and advanced training strategies, this method addresses the challenge of interpreting complex signals in cardiology.

The use of physiological models may ensure that the synthesized measurement data is consistent with known anatomical and physiological principles. This consistency can improve the reliability and accuracy of the foundation model's training process, which in turn may lead to better performance when the model is applied to real-world clinical data for disease diagnosis or severity assessment.

According to various examples, the computer-implemented method further comprises generating further synthesized measurement data of at least one physiological parameter based on previously synthesized measurement data and one or more functional models associated with medical devices suitable for obtaining measurements of that physiological parameter. The text data further describes simulated characteristics of these functional models linked to the medical devices. This approach allows the foundation model to be trained using both the expanded text data and the further synthesized measurement data.

By this, realistic noise and artifacts may be included into the synthesized signals. By incorporating functional models of medical devices, the synthesized measurement data becomes more representative of real-world scenarios, where measurements are often affected by various sources of interference and imperfections inherent in clinical settings. This enhanced realism can improve the robustness and accuracy of the foundation model when applied to actual clinical data.

The inclusion of simulated characteristics from functional models ensures that the training data reflects a wide range of potential measurement conditions. This diversity helps the model generalize better across different medical devices and operational environments, ultimately leading to more reliable and accurate interpretations of physiological signals in various clinical contexts.

According to various examples, the foundation model comprises a first encoder and a second encoder. The training of the foundation model includes determining one or more text embeddings from the text data using the first encoder and one or more measurement embeddings from the synthesized measurement data using the second encoder. The parameter values of the foundation model are then updated based on an association between these text embeddings and measurement embeddings.

In this context, "embedding" may refer to a vector representation that captures the essential features of the data, whether it is textual or numerical measurements. Each encoder may perform an "encoding" function that may be the process through which these embeddings are generated by transforming raw data into a more compact and meaningful form.

This approach may optionally use contrastive learning and/or language models. Contrastive learning techniques may enhance the model's ability to distinguish between different data points by comparing and contrasting their embeddings, thereby improving the quality of learned representations. Additionally, incorporating language models can further refine text embeddings by capturing contextual relationships within the text, leading to more informative associations with measurement embeddings.

The use of the two encoders may allow for specialized processing of each data type, ensuring that both textual descriptions and numerical measurements are effectively represented in their respective embedding spaces. By updating model parameters based on these associated embeddings, the foundation model becomes more adept at linking textual information with numerical measurements, ultimately enhancing its performance in interpreting physiological signals for disease classification and other clinical applications.

According to various examples, the synthesized measurement data or the (noisy) further synthesized measurement data is associated with multiple physiological parameters. Each set of measurement data corresponding to one of these physiological parameters is directed to a separate branch within the second encoder.

In this context, a "branch" may refer to a distinct processing pathway within the second encoder, dedicated to handling measurement data from specific physiological parameters. This architecture may allow for specialized and tailored processing of each parameter's data.

By segregating the measurement data into separate branches based on physiological parameters, the model can more effectively capture parameter-specific features. This separation may enhance the model's ability to learn discriminative representations by leveraging contrastive learning techniques, which may improve the distinction between different biological signals.

According to various examples, the computer-implemented method involves updating the parameter values of the foundation model based on a contrastive loss between the one or more text embeddings and the one or more measurement embeddings. This technique leverages the concept of contrastive learning, which is a training objective that encourages similar embeddings to be closer together while pushing dissimilar embeddings further apart.

A "contrastive loss" in this context may refer to a mathematical function used during training to measure the difference between embeddings of similar and dissimilar pairs, guiding the model to refine its parameters for better representation learning. This may enhance the ability of the foundation model to capture shared information across text and measurement data, improving its overall performance in tasks such as disease classification.

Additionally, according to various examples, the foundation model may further include a decoder that generates further text data based on the one or more text embeddings and the one or more measurement embeddings. Generally, a decoder may relate to a machine-learning model, for example a neural network (or part of one), that transforms encoded latent representations back into a human-interpretable format, such as text, images, or signals.

The parameter values of the foundation model are then updated further based on a comparison between the original text data and the generated further text data from the decoder. This technique allows the model to refine its understanding of both text and measurement information by creating coherent and contextually relevant text outputs. The decoder may enable the generation of meaningful text that aligns well with the input embeddings.

According to various examples, the computer-implemented method further comprises fine-tuning the foundation model based on manually created text data describing one or more medical conditions associated with the anatomical target region of a patient and clinical measurement data of the at least one physiological parameter associated with the anatomical target region of the patient. This process involves adjusting the model's parameters using tailored datasets that include specific descriptions of medical conditions and corresponding clinical measurements for the target region, enabling the model to better understand the relationship between these variables.

Manually created text data may be provided in clinical environments by a physician examining a patient while corresponding measurement data are recorded.

Fine-tuning may involve further training this model on specific, manually created datasets to enhance its performance for particular tasks, such as disease diagnosis or patient monitoring.

This technique may adapt the foundation model to recognize patterns within medical conditions and their associated physiological parameters. By leveraging manually created text data that describes medical conditions in detail, the model may gain a deeper understanding of clinical terminology and context. Additionally, incorporating clinical measurement data may ensure that the model can correlate specific physiological indicators with the described medical conditions, improving its diagnostic accuracy and decision-making capabilities.

This fine-tuning process may also enhance the model's ability to generalize across diverse patient populations by exposing it to a wide range of scenarios and examples. Furthermore, the use of manually created datasets may allow for high-quality training data that is less prone to errors or biases compared to automatically generated data, thereby improving the reliability and robustness of the foundation model in clinical applications.

According to various examples, the anatomical target region comprises at least one of an aortic valve, a mitral valve, a pulmonary valve, and a tricuspid valve. The at least one physiological parameter includes at least one of a blood velocity, a left ventricular outflow tract diameter, a transvalvular pressure gradient, and a left ventricular volume. The blood velocity may be a blood velocity at the corresponding valve, e.g. a blood velocity at an aortic valve annulus. Additionally, the medical conditions involve the presence of at least one of stenosis, regurgitation, prolapse, and atresia.

In this context, the anatomical target region refers to specific heart valves critical for maintaining proper blood flow. Physiological parameters may be measurable indicators of cardiac functions that help to assess valve performance. The medical conditions listed represent common valvular pathologies, each with distinct characteristics affecting heart function differently.

Focusing on these specific components may enhance model accuracy in identifying and monitoring heart valve-related conditions. By concentrating on relevant physiological metrics and well-defined medical conditions, the model may improve its ability to detect anomalies such as stenosis or regurgitation. Moreover, this targeted technique may ensure that the model is well-suited for analyzing data from diverse patient populations with various valve-related issues. The defined relevance of the parameters and conditions may facilitate a more accurate correlation between physiological measurements and potential valvular pathologies.

A further aspect of the present disclosure relates to a computer-implemented method for establishing a diagnosis of a disease associated with a heart valve. The method involves obtaining a series of medical images that depict the heart valve. For example, the series of medical images may be a time series of medical images depicting the heart value over time. In some examples, such as Doppler echocardiography, the series of medical images may be a single medical image with multiple time points. Based on this series, measurement data of at least one physiological parameter associated with the heart valve is determined. Generally, the data to be measured may be a temporal signal and any kind of images or data that are used clinically to represent or measure that signal may be taken as input. Using the second encoder of the foundation model described above, measurement embeddings are generated from the measurement data. Finally, a machine-learning model is utilized to establish the diagnosis of the disease based on these measurement embeddings. In some examples, the machine-learning model may include or may be based on the decoder of the foundation model. In some examples, the machine-learning model may be implemented separately and independent from foundation model.

In this context, medical images refer to visual representations of the heart valve captured at different points in time, allowing for the analysis of its dynamic behavior and functional changes. The medical images may be captured by ultrasound or magnetic resonance imaging (MRI). Physiological parameters are specific measurable indicators that provide insights into the valve's performance and health status. Measurement embeddings represent a compressed and meaningful representation of these physiological parameters, derived through encoding techniques.

Thus, temporal changes in heart valve function may be analyzed, leveraging the power of machine-learning models to extract insightful features from medical data. By utilizing measurement embeddings, the method can capture complex patterns in physiological parameters that are critical for accurate diagnosis. This approach enhances diagnostic accuracy and reliability.

According to various examples, establishing the diagnosis of the disease is based on text embeddings associated with the measurement embeddings. Text embeddings may be vector representations of textual information that capture semantic relationships within the text. By integrating text embeddings that are closest to the measurement embeddings derived from medical images, the method gains the ability to leverage both textual and numerical data for a more comprehensive analysis. For example, a disease diagnosis can be performed using the text embeddings that are closest to the measurement embeddings, obtained for example by zero-shot evaluation of the model.

In further examples, further text data from one or more text embeddings and measurement embeddings may be generated. Establishing the diagnosis of the disease may be based on the further text data. This involves creating descriptive summaries or integrating multiple sources of evidence to aid in the diagnostic process. By combining text and measurement embeddings, the method can generate new text data that provides a more detailed and integrated analysis.

This analysis may support a physician in determining a diagnosis.

The integration of both textual and numerical data allows the system to perform classification tasks using multiple input signals and assess consistency between these inputs by measuring the distance between their embeddings. If inconsistencies are detected, the system can generate warnings or notifications, further enhancing the reliability of the diagnostic process. For example, based on the warnings or notifications, the physician may change the examination setup or procedure.

The diagnostic process may also be based on this scenario if multiple input signals are provided. In this case, the diagnostic process may use the result obtained from processing the multiple inputs as input.

According to various examples, the machine-learning model is a classifier. In other words, the computer-implemented method employs a classifier as the machine-learning model. The classifier, in this context, may refer to a type of machine-learning model designed to predict categorical labels or classes from input data.

The use of a classifier may enable the method to categorize inputs into distinct categories based on patterns learned during training. This capability enhances the overall functionality by providing clear and interpretable outputs. Furthermore, the classifier may improve the accuracy and reliability of predictions or decisions made by the system. By leveraging well-defined categories, the method ensures that outputs are not only precise but also consistent with the expected classifications derived from the training data.

In the following, techniques with reference to foundation models will be described. Generally, a foundation model is a large-scale artificial intelligence model trained on vast amounts of data and designed to serve as a base for a variety of downstream tasks. These models, often built using deep learning techniques, can be adapted for specific applications through fine-tuning or prompting. Foundation Models can learn from diverse and large datasets, often sourced from the internet, books, images, and more. Unlike traditional AI models designed for narrow tasks, foundation models can be adapted to multiple applications, such as language translation, image generation, and medical diagnosis. Once trained, they can be fine-tuned for specialized tasks with significantly less data and computational resources than training a model from scratch.

Artificial neural networks - for example large-scale deep learning architectures - may be the basis of foundation models. These models may typically be implemented using transformer-based neural networks, which excel at handling vast amounts of sequential data (e.g., text, images, audio).

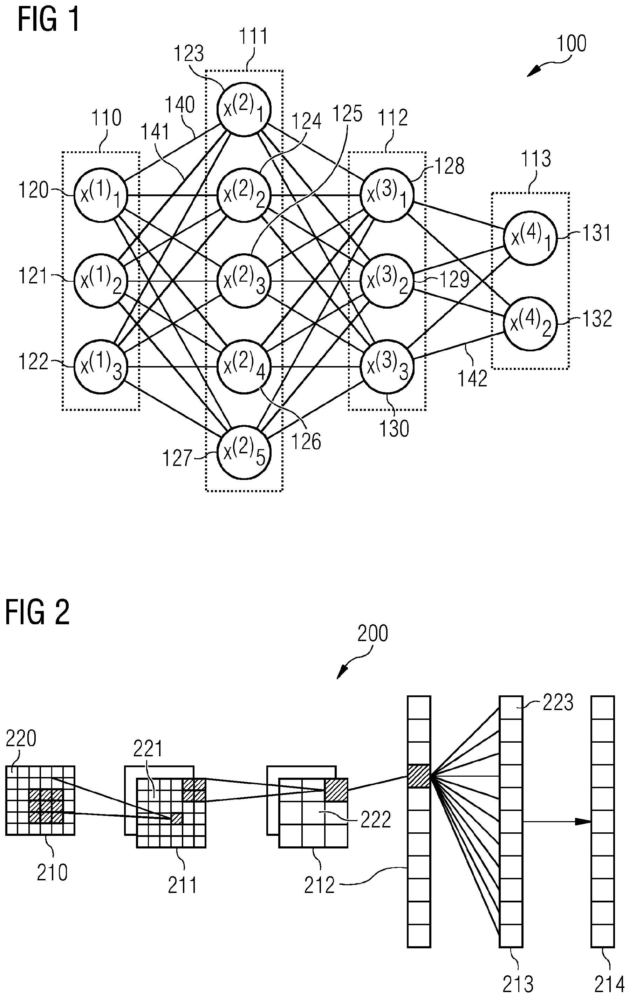

Fig. 1 displays an embodiment of an artificial neural network 100. Alternative terms for "artificial neural network" are "neural network", "artificial neural net" or "neural net".

The artificial neural network 100 comprises nodes 120, ..., 132 and edges 140, ..., 142, wherein each edge 140, ..., 142 is a directed connection from a first node 120, ..., 132 to a second node 120, ..., 132. In general, the first node 120, ..., 132 and the second node 120, ..., 132 are different nodes 120, ..., 132, it is also possible that the first node 120, ..., 132 and the second node 120, ..., 132 are identical. For example, in Fig. 1 the edge 140 is a directed connection from the node 120 to the node 123, and the edge 142 is a directed connection from the node 130 to the node 132. An edge 140, ..., 142 from a first node 120, ..., 132 to a second node 120, ..., 132 is also denoted as "ingoing edge" for the second node 120, ..., 132 and as "outgoing edge" for the first node 120, ..., 132.

In this embodiment, the nodes 120, ..., 132 of the artificial neural network 100 can be arranged in layers 110, ..., 113, wherein the layers can comprise an intrinsic order introduced by the edges 140, ..., 142 between the nodes 120, ..., 132. In particular, edges 140, ..., 142 can exist only between neighboring layers of nodes. In the displayed embodiment, there is an input layer 110 comprising only nodes 120, ..., 122 without an incoming edge, an output layer 113 comprising only nodes 131, 132 without outgoing edges, and hidden layers 111, 112 in-between the input layer 110 and the output layer 113. In general, the number of hidden layers 111, 112 can be chosen arbitrarily. The number of nodes 120, ..., 122 within the input layer 110 usually relates to the number of input values of the neural network, and the number of nodes 131, 132 within the output layer 113 usually relates to the number of output values of the neural network.

In particular, a (real) number can be assigned as a value to every node 120, ..., 132 of the neural network 100. Here, $x^{(n)}_i$ denotes the value of the i-th node 120, ..., 132 of the n-th layer 110, ..., 113. The values of the nodes 120, ..., 122 of the input layer 110 are equivalent to the input values of the neural network 100, the values of the nodes 131, 132 of the output layer 113 are equivalent to the output value of the neural network 100. Furthermore, each edge 140, ..., 142 can comprise a weight being a real number, in particular, the weight is a real number within the interval [-1, 1] or within the interval [0, 1]. Here, $w^{(m,n)}_{i,j}$ denotes the weight of the edge between the i-th node 120, ..., 132 of the m-th layer 110, ..., 113 and the j-th node 120, ..., 132 of the n-th layer 110, ..., 113. Furthermore, the abbreviation $w^{(n)}_{i,j}$ is defined for the weight $w^{(n,n+1)}_{i,j}$.

In particular, to calculate the output values of the neural network 100, the input values are propagated through the neural network. In particular, the values of the nodes 120, ..., 132 of the (n+1)-th layer 110, ..., 113 can be calculated based on the values of the nodes 120, ..., 132 of the n-th layer 110, ..., 113 by

$$x_j^{(n+1)} = f\left(\sum_i x_i^{(n)} \cdot w_{i,j}^{(n)}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 110 are given by the input of the neural network 100, wherein values of the first hidden layer 111 can be calculated based on the values of the input layer 110 of the neural network, wherein values of the second hidden layer 112 can be calculated based in the values of the first hidden layer 111, etc.

In order to set the values $w^{(m,n)}_{i,j}$ for the edges, the neural network 100 has to be trained using training data. In particular, training data comprises training input data and training output data (denoted as $t_i$). For a training step, the neural network 100 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 100 (backpropagation algorithm). In particular, the weights are changed according to

$$w'^{(n)}_{i,j} = w_{i,j}^{(n)} - \gamma \cdot \delta_j^{(n)} \cdot x_i^{(n)}$$

wherein $\gamma$ is a learning rate, and the numbers $\delta^{(n)}_j$ can be recursively calculated as

$$\delta_j^{(n)} = \left(\sum_k \delta_k^{(n+1)} \cdot w_{j,k}^{(n+1)}\right) \cdot f'\left(\sum_i x_i^{(n)} \cdot w_{i,j}^{(n)}\right)$$

based on $\delta^{(n+1)}_j$, if the (n+1)-th layer is not the output layer, and

$$\delta_j^{(n)} = \left(x_k^{(n+1)} - t_j^{(n+1)}\right) \cdot f'\left(\sum_i x_i^{(n)} \cdot w_{i,j}^{(n)}\right)$$

if the (n+1)-th layer is the output layer 113, wherein f' is the first derivative of the activation function, and $y^{(n+1)}_j$ is the comparison training value for the j-th node of the output layer 113.

Fig. 2 displays an embodiment of a convolutional neural network (CNN) 200. In the displayed embodiment, the convolutional neural network comprises 200 an input layer 210, a convolutional layer 211, a pooling layer 212, a fully connected layer 213 and an output layer 214. Alternatively, the convolutional neural network 200 can comprise several convolutional layers 211, several pooling layers 212 and several fully connected layers 213, as well as other types of layers. The order of the layers can be chosen arbitrarily, usually fully connected layers 213 are used as the last layers before the output layer 214.

In particular, within a convolutional neural network 200 the nodes 220, ..., 224 of one layer 210, ..., 214 can be considered to be arranged as a d-dimensional matrix or as a d-dimensional image. In particular, in the two-dimensional case the value of the node 220, ..., 224 indexed with i and j in the n-th layer 210, ..., 214 can be denoted as $x^{(n)}[i,j]$. However, the arrangement of the nodes 220, ..., 224 of one layer 210, ..., 214 does not have an effect on the calculations executed within the convolutional neural network 200 as such, since these are given solely by the structure and the weights of the edges.

In particular, a convolutional layer 211 is characterized by the structure and the weights of the incoming edges forming a convolution operation based on a certain number of kernels. In particular, the structure and the weights of the incoming edges are chosen such that the values $x^{(n)}_k$ of the nodes 221 of the convolutional layer 211 are calculated as a convolution $x^{(n)}_k = K_k * x^{(n-1)}$ based on the values $x^{(n-1)}$ of the nodes 220 of the preceding layer 210, where the convolution * is defined in the two-dimensional case as

$$x_k^{(n)}[i,j] = \left(K_k * x^{(n-1)}\right)[i,j] = \sum_{i'}\sum_{j'} K_k[i',j'] \cdot x^{(n-1)}[i-i', j-j'].$$

Here the k-th kernel $K_k$ is a d-dimensional matrix (in this embodiment a two-dimensional matrix), which is usually small compared to the number of nodes 220, ..., 224 (e.g. a 3x3 matrix, or a 5x5 matrix). In particular, this implies that the weights of the incoming edges are not independent but chosen such that they produce said convolution equation. In particular, for a kernel being a 3x3 matrix, there are only 9 independent weights (each entry of the kernel matrix corresponding to one independent weight), irrespectively of the number of nodes 220, ..., 224 in the respective layer 210, ..., 214. In particular, for a convolutional layer 211 the number of nodes 221 in the convolutional layer is equivalent to the number of nodes 220 in the preceding layer 210 multiplied with the number of kernels.

If the nodes 220 of the preceding layer 210 are arranged as a d-dimensional matrix, using a plurality of kernels can be interpreted as adding a further dimension (denoted as "depth" dimension), so that the nodes 221 of the convolutional layer 221 are arranged as a (d+1)-dimensional matrix. If the nodes 220 of the preceding layer 210 are already arranged as a (d+1)-dimensional matrix comprising a depth dimension, using a plurality of kernels can be interpreted as expanding along the depth dimension, so that the nodes 221 of the convolutional layer 221 are arranged also as a (d+1)-dimensional matrix, wherein the size of the (d+1)-dimensional matrix with respect to the depth dimension is by a factor of the number of kernels larger than in the preceding layer 210.

The advantage of using convolutional layers 211 is that spatially local correlation of the input data can exploited by enforcing a local connectivity pattern between nodes of adjacent layers, in particular by each node being connected to only a small region of the nodes of the preceding layer.

In the displayed embodiment, the input layer 210 comprises 36 nodes 220, arranged as a two-dimensional 6x6 matrix. The convolutional layer 211 comprises 72 nodes 221, arranged as two two-dimensional 6x6 matrices, each of the two matrices being the result of a convolution of the values of the input layer with a kernel. Equivalently, the nodes 221 of the convolutional layer 211 can be interpreted as arranges as a three-dimensional 6x6x2 matrix, wherein the last dimension is the depth dimension.

A pooling layer 212 can be characterized by the structure and the weights of the incoming edges and the activation function of its nodes 222 forming a pooling operation based on a non-linear pooling function f. For example, in the two-dimensional case the values $x^{(n)}$ of the nodes 222 of the pooling layer 212 can be calculated based on the values $x^{(n-1)}$ of the nodes 221 of the preceding layer 211 as

$$x^{(n)}[i,j] = f(x^{(n-1)}[id_1, jd_2], \ldots, x^{(n-1)}[id_1+d_1-1, jd_2+d_2-1])$$

In other words, by using a pooling layer 212 the number of nodes 221, 222 can be reduced, by replacing a number $d_1 \cdot d_2$ of neighboring nodes 221 in the preceding layer 211 with a single node 222 being calculated as a function of the values of said number of neighboring nodes in the pooling layer. In particular, the pooling function f can be the max-function, the average or the L2-Norm. In particular, for a pooling layer 212 the weights of the incoming edges are fixed and are not modified by training.

The advantage of using a pooling layer 212 is that the number of nodes 221, 222 and the number of parameters is reduced. This leads to the amount of computation in the network being reduced and to a control of overfitting.

In the displayed embodiment, the pooling layer 212 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 213 can be characterized by the fact that a majority, in particular, all edges between nodes 222 of the previous layer 212 and the nodes 223 of the fully-connected layer 213 are present, and wherein the weight of each of the edges can be adjusted individually.

In this embodiment, the nodes 222 of the preceding layer 212 of the fully-connected layer 213 are displayed both as two-dimensional matrices, and additionally as non-related nodes (indicated as a line of nodes, wherein the number of nodes was reduced for a better presentability). In this embodiment, the number of nodes 223 in the fully connected layer 213 is equal to the number of nodes 222 in the preceding layer 212. Alternatively, the number of nodes 222, 223 can differ.

Furthermore, in this embodiment the values of the nodes 224 of the output layer 214 are determined by applying the Softmax function onto the values of the nodes 223 of the preceding layer 213. By applying the Softmax function, the sum of the values of all nodes 224 of the output layer is 1, and all values of all nodes 224 of the output layer are real numbers between 0 and 1. In particular, if using the convolutional neural network 200 for categorizing input data, the values of the output layer can be interpreted as the probability of the input data falling into one of the different categories.

A convolutional neural network 200 can also comprise a ReLU (acronym for "rectified linear units") layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are $f(x) = \max(0,x)$, the tangent hyperbolics function or the sigmoid function.

In particular, convolutional neural networks 200 can be trained based on the backpropagation algorithm. For preventing overfitting, methods of regularization can be used, e.g. dropout of nodes 220, ..., 224, stochastic pooling, use of artificial data, weight decay based on the L1 or the L2 norm, or max norm constraints.

The training and use of foundation models in the medical field to aid diagnosis are described in more detail below. Although the techniques presented relate primarily to cardiac physiology, they are also applicable to other anatomical regions and organs, e.g. kidney, liver, intestine or brain.

Foundation models for cardiac physiology signals have the potential to improve cardiac signal analysis by three key advantages:

1. Trained on large amounts of data to learn generalizable representations.
2. Leverage synthetically generated signals based on physiological models, for a wide range of conditions and including measurement noise.
3. Leverage synthetically generated textual descriptions for multimodal representation learning.

The foundation model may be trained to learn the association between signals and rich descriptive text. The text describes the signal in terms of the underlying physiological phenomenon, the condition (normal physiology vs. pathology) and the measurement quality.

To provide a rich data set of training samples, physiological models can be utilized to generate realistic temporal signals associated to the functioning of the heart. Several such physiological models have been extensively studied over the years and are the basis for interpretation of clinical measurements, see for example Caro, C. G., T. J. Pedley, R. C. Schroter, W. A. Seed, and K. H. Parker: The Mechanics of the Circulation; 2nd ed. Cambridge University Press, 2011. Together with the generation of realistic temporal signals, the physiological models may be used to produce descriptive text, including qualitative or quantitative accounts of the characteristics of the physiological model used, and the values of its parameters (including presence of noise or artifacts).

The training set may thus be made of paired data: a realistic temporal signal and the corresponding descriptive text. Using multimodal contrastive learning, a foundation model may be trained to learn the semantic link between signals and their textual explanation.

After training, the foundation model can be used in multiple ways:

- Zero-shot evaluation: given a signal and multiple possible text labels, pick the most appropriate text description based on the distance of the corresponding embeddings.
- Linear probing, e.g. for classification: add one or more layers after the signal encoder to perform downstream tasks such as disease classification. The encoder is frozen, while the additional layers are fine-tuned using a dedicated training set.
- Full fine-tuning: analogous to linear probing, but the entire set of weights (encoder + additional layers) can be updated during fine-tuning.

As an exemplary application, the use of the foundation model for the classification of mitral regurgitation may be considered. According to the publication "Recommendations for Noninvasive Evaluation of Native Valvular Regurgitation" by Zoghbi, William A., David Adams, Robert O. Bonow, Maurice Enriquez-Sarano, Elyse Foster, Paul A. Grayburn, Rebecca T. Hahn, et al., Journal of the American Society of Echocardiography 30, no. 4 (April 2017): 303-71, chronic mitral regurgitation can be assessed by echocardiography. Such assessment requires the integrated evaluation of multiple measured signals. It is also crucial to recognize the quality of the available measurements - if quality is insufficient, alternative quantification strategies or imaging modalities should be utilized.

In the following, details with respect to the generation of a training set, a training strategy, and an application to valve disease assessment are disclosed.

An example of training set generation is described below with reference to cardiac physiology.

The human heart is a complex organ where various phenomena occur, and many of these can be measured as temporal signals. For instance, the electrical activity that coordinates the heartbeat is captured by electrocardiograms (ECG), while the mechanical actions, such as the opening and closing of heart valves and the blood flow within the heart chambers, can be measured by ultrasound or magnetic resonance imaging (MRI). The underlying physics of these phenomena, including blood velocity and pressure, are well understood and can be modeled using the laws of physics.

By leveraging physiological models based on the same physics laws, it's possible to simulate specific cardiac phenomena and generate the corresponding temporal signals. These models are governed by a set of parameters that can be adjusted to reflect changes in the underlying physiological phenomena, such as simulating the presence of valvular stenosis and its effects on the blood pressure in the ventricles and arteries. Additionally, the measurement devices themselves can be modeled by simulating the physics of the diagnostic tools, like ultrasound. This includes the ability to introduce realistic noise and artifacts into the simulated signals, thereby creating a more accurate representation of what would be observed in actual heart measurements.

Given that physiological models are governed by a multitude of parameters, each generated signal can be accompanied by descriptive text that reports the value and meaning of these parameters. This descriptive text serves as a key to interpreting the signal, providing insights into the specific conditions being simulated. For example, if a physiological model simulates the effect of valvular stenosis, the descriptive text will detail the name of the condition itself (e.g., aortic stenosis), as well as parameters that have been adjusted to reflect this condition, such as increased pressure gradients across the valve or reduced valve area.

In an embodiment, the descriptive text may include one or more of:

- The name of the physical quantity being measured (e.g., blood velocity).
- The time span over which it is measured (e.g., one / multiple heart beats).
- The range of values (e.g., between 0 and 50 cm/s).
- The location of the measurement (e.g., at the level of the aortic valve annulus).
- The condition being simulated (e.g., aortic stenosis).
- The measurement device being simulated (e.g., pulsed-wave Doppler).
- The settings of the device (e.g., frame rate, gain level).
- Any added source of noise (e.g., aliasing, blooming).

The term "blooming" may relate to an imaging artifact that can affect the accuracy of diagnoses in heart valve diseases by altering the appearance of structures on medical images. See also the publication of Le, Huong T., Nicholas Hangiandreou, Robert Timmerman, Mark J. Rice, W. Brit Smith, Lori Deitte, and Gregory M. Janelle. "Imaging Artifacts in Echocardiography." Anesthesia & Analgesia 122, no. 3 (March 2016): 633-46.

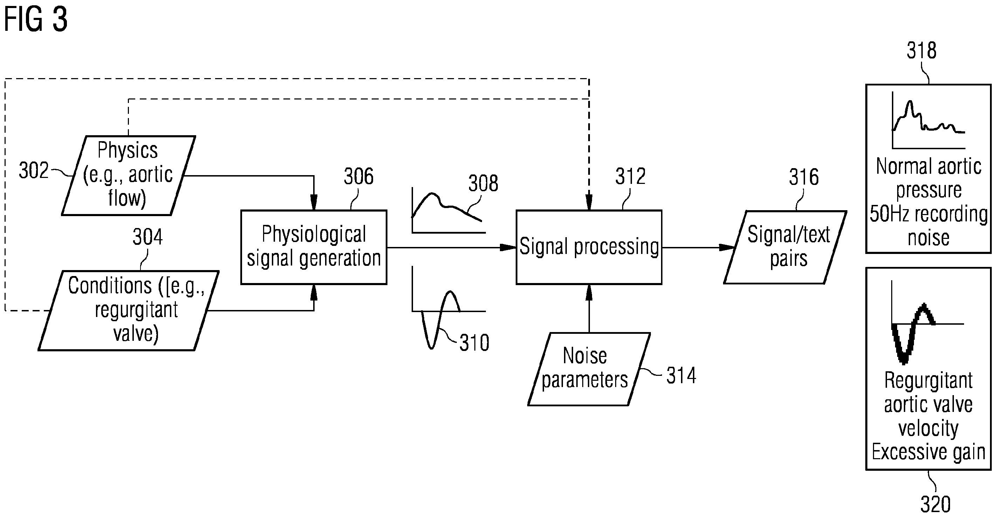

A schematic representation of the generation of training samples is presented in FIG. 3.

In an embodiment, training samples (including signal and descriptive text) may be extracted from documents such as books about cardiac physiology. Signals can be extracted from plots in the book, via plot digitization. Descriptive text can be extracted from the plot caption or the document text connected to the plot. See, for example, Yu, Han, Peikun Guo, and Akane Sano. "ECG Semantic Integrator (ESI): A Foundation ECG Model Pretrained with LLM-Enhanced Cardiological Text." arXiv, May 26, 2024. In some examples, training samples may be generated by physiological models set up to produce a desired output, e.g. corresponding to a desired medical condition.

For example, a physiological model may be set up to produce a desired output. For example, numerical parameters or logical conditions may be set up in the model's software producing the physical signals, see box 302. These numerical parameters and logical conditions may represent medical condition, see box 304, and may be converted to text since their meaning may be known to the operator. Based on this, physiological signals 308, 310 may be generated, see box 306, e.g. by means of a computation unit. In the example of FIG. 3, physiological signal 308 may represent an aortic pressure over time and physiological signal 310 may represent a regurgitant aortic valve velocity over time. Signals 308 and 310 may be generated based on physical laws implemented by the physiological model.

In some examples, physical signals, see box 302, such as an aortic flow over time may be extracted from corresponding plots in a publication such as a book, a scientific paper or the Internet. Corresponding text representing medical conditions such as valve regurgitation, see box 304, may be also extracted from the publication. Based on the physical signals, physiological signals 308, 310 may be generated, see box 306, e.g. by means of a computation unit. In the example of FIG. 3, physiological signal 308 may represent an aortic pressure over time and physiological signal 310 may represent a regurgitant aortic valve velocity over time. Signals 308 and 310 may be generated based on physical laws, a physiological model or use of machine-learning techniques based on the physical signals, e.g. the aortic flow, as an input.

In box 312, noise is added to the physiological signals 308, 310. Noise parameters 314 may be provided and applied on the physiological signals 308, 310 considering typical noise parameters associated with the medical devices used for acquiring the physical signals, see dashed arrows in FIG. 3. As a result, signal/text pairs may be achieved, each pair including a (noisy) signal and corresponding text as an output, see box 316. Exemplary pairs are pair 318 including a signal representing normal aortic pressure with 50 Hz recording noise and corresponding text, and pair 320 including a signal representing a regurgitant aortic valve velocity excessive gain and corresponding text. As illustrated, the signal of pair 320 may include noise/ripple.

A large variety of pairs including a signal and corresponding descriptive text may be used in a training system for training a foundation model. A corresponding exemplary training strategy is outlined below.

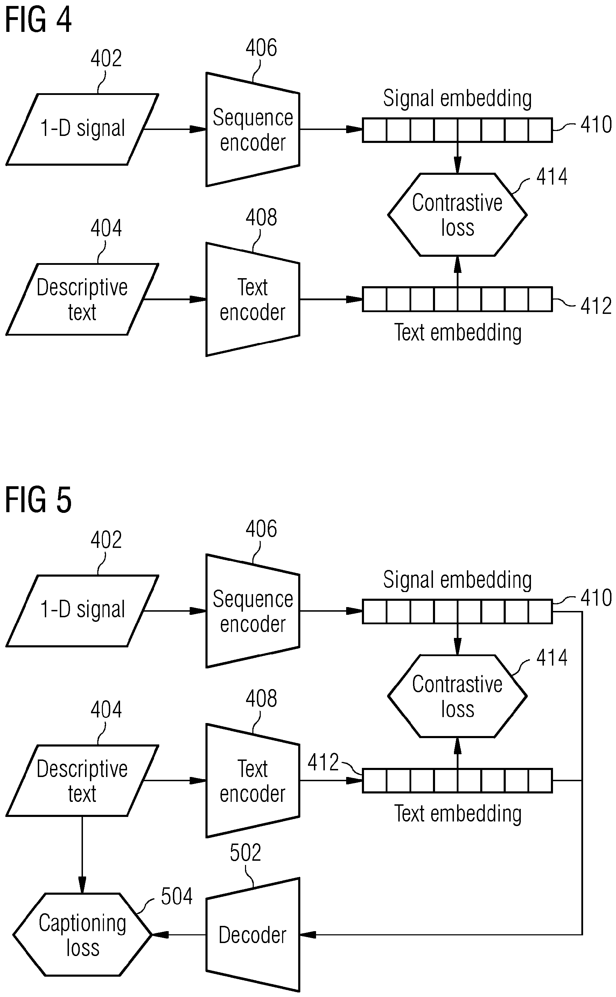

The foundation model can be trained with techniques used for multimodal contrastive learning. FIG. 4 illustrates an example. Each input source, i.e. signal input 402 and descriptive text input 404, may be processed by a dedicated encoder, i.e. the input signal 402 by a sequence encoder 406 and the descriptive text 404 by a text encoder 408. The text input 404 may correspond to the medical conditions of box 304. The signal input 402 may correspond to the physiological signals 308, 310, optionally including the added noise. Both encoders may be part of the foundation model and may produce embeddings in a unified space. The signal input 402 may be the noisy signal of one of the pairs 318, 320 generated according to FIG. 3. The descriptive text 404 may be the descriptive text of the same pair. The sequence encoder 406 produces a signal embedding 410 and the text encoder 408 produces a text embedding 412. Contrastive learning can then be applied on the foundation model to learn the relationship between text encoding and signal encoding.

In this context, an encoder may relate to a neural network (e.g. part of the foundation model) that transforms raw input data (e.g., text, images, or signals) into a lower-dimensional latent representation. The text/signal embedding may be considered as a dense, learned representation of the encoded data (e.g., words, images, or signals) in, for example, a continuous vector space, preserving meaningful relationships. The contrastive learning may be a self-supervised learning technique where the foundation model learns to distinguish between similar and dissimilar data points by maximizing similarity for related pairs and minimizing it for unrelated pairs. A contrastive loss may be a loss function used in contrastive learning to encourage similar data points to have closer embeddings while pushing apart dissimilar ones. Hence, in the example of FIG. 4, a contrastive loss 414 may be determined based on the signal embedding 410 and the text embedding 412. The foundation model, in particular the encoders 406, 408, may be trained to minimize the contrastive loss 414.

The signal 402 may be presented as a sequence of numbers. A 1-D encoder such as ConvNext can be used for encoding the signal 402. The term ConvNext is derived from "Convolutional Network Next" (indicating the next generation of convolutional networks). For encoding the descriptive text 404, a language model such as "Bidirectional Encoder Representations from Transformers" (BERT) can be used.

In an embodiment, an additional loss term can be added to the contrastive loss, see also Yu, Han, Peikun Guo, and Akane Sano. "ECG Semantic Integrator (ESI): A Foundation ECG Model Pretrained with LLM-Enhanced Cardiological Text." arXiv, May 26, 2024.4. FIG. 5 illustrates more details. The two embeddings 410, 412 can be fed to a decoder 502 which produces a sequence of tokens as aligned as possible to the original descriptive text 404. The foundation model with two encoders 406, 408 and one dual-input decoder 502 can be trained to minimize a captioning loss 504 measuring the misalignment between the generated tokens and original tokens, i.e. between the descriptive text from the decoder 502 and the descriptive text 404. This version of the foundation model can be used to produce accurate description of the condition associated with the signal, based on the associated text embedding selected by the foundation model.

The foundation model thus trained can be used in a providing system to aid in the diagnosis of a disease, such as the assessment of valve disease.

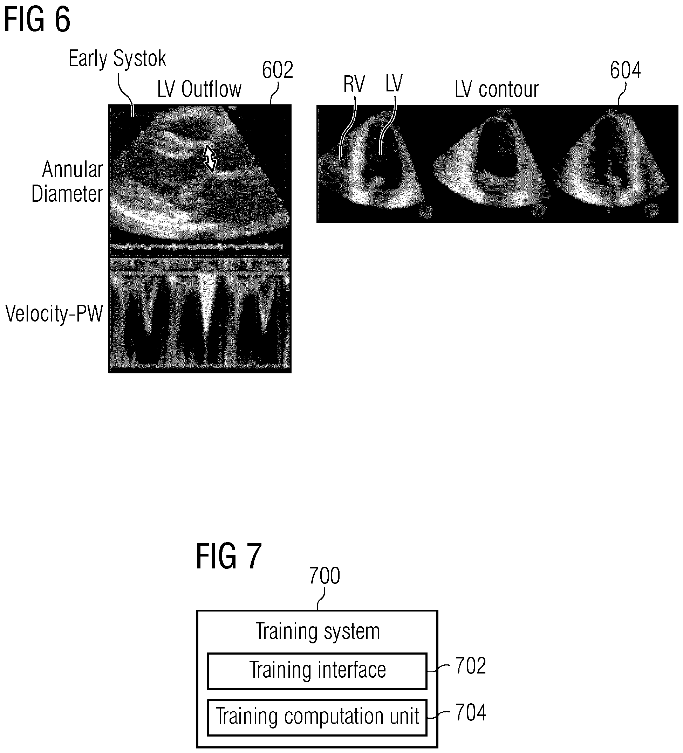

For example, as illustrated in FIG. 6, for the assessment of mitral regurgitation, multiple echocardiography images may be acquired, including in particular Pulsed Wave (PW) Doppler in the aortic annulus, see 602, and movies of the moving ventricles and left ventricular (LV) tract, see 604. Echocardiography, also known as cardiac ultrasound, is the use of ultrasound to examine the heart. PW Doppler is an ultrasound technique used in echocardiography to assess blood flow. It may provide information on both the direction and speed of blood flow at specific locations within the heart or vessels.

Each of these images can be processed with AI-based models for segmentation and tracking of the visible structures, for instance:

- Automatic delineation of blood velocity in PW Doppler measurements at the level of the aortic valve annulus,
- Automatic delineation of the LV endocardial wall in multiple apical views,
- Automatic delineation of the LV outflow tract (LVOT) in parasternal long axis views.

By extracting the measurements on each temporal frame of an acquisition spanning one or more heart beats, a digitized one-dimensional signal can be extracted from each of the mentioned images. Each signal represents the temporal evolution of a quantity of interest, corresponding to an underlying physical phenomenon, e.g.:

- Blood velocity over time at the aortic valve annulus,
- LVOT diameter over time,
- LV volume over time.

For each digitized signal, the trained foundation model may produce a descriptive text based on the examples produced by the physiological models used to create the training set. The descriptive text may contain information about

- Quality of the signal,
- Values of the model parameters which would produce a similar signal,
- Underlying conditions.

In some examples, the foundation model may be further adapted to a classification task by linear probing or full fine-tuning. In this scenario, a small number of real data sets acquired from subjects with known disease condition (e.g., mild/moderate/severe mitral valve regurgitation) are used to fine-tune the foundation model. The resulting fine-tuned model can perform disease severity classification based on the input signal.

In some examples, a classifier may be trained similarly to the previous scenario, but taking as input the signal embeddings produced by the original foundation model on the three aforementioned signals (blood velocity in the aortic annulus, diameter of the LVOT, volume of the LV).

In a further example, in addition to performing a classification task based on multiple signals as input, the system may assess the consistency between the input signals. The embeddings of the text descriptions associated with the input signals may be evaluated, by computing the mutual distance. Text embeddings are expected to be close to each other in the latent space, if the signals are consistently associated with similar conditions (e.g., presence or absence of valvular disease). Distances greater than a given threshold could be taken as a marker for inconsistent input signals, which would lead to a warning message being produced, or other user notification strategies.

The above-described techniques for generation of a training set and the training strategy for training a foundation model may be implemented in a training system 700 illustrated in FIG. 7. The training system 700 comprises a training interface 702 configured for obtaining text data describing one or more medical conditions and a training computation unit 704. The training interface 702 may further be configured for obtaining physical signals, for example a plot of a physical signal, associated with the text data. If physical signals are obtained from plots etc, then it may be possible to just use these without generating physiological signals with the physiological model. For example, the plot of the physical signal may indicate a physical observable captured at an organ having the medical conditions described in the text data. The training interface 702 may comprise, for example a camera for capturing images of corresponding text data and plots of physical signals from a book or printed document, or the training interface 702 may comprise an interface to a database of a library or the Internet providing corresponding medical information. The training computation unit 704 may comprise, for example, one or more processing circuits such as a computer. Such circuits and other electrical devices may be combined with each other and/or separated in any manner based on the particular type of electrical implementation that is desired. It is recognized that any circuit or other electrical device disclosed herein may include any number of microcontrollers, a central processing unit (CPU), a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

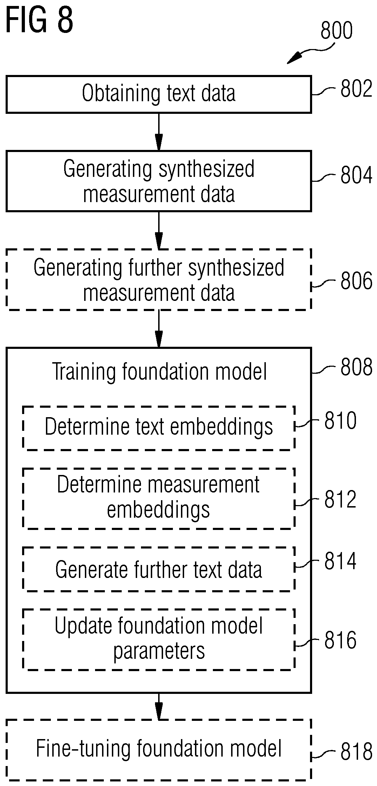

For example, the training computation unit 704 may be configured to execute the method steps 802 to 818 of method 800 illustrated in FIG. 8.

In step 802, text data describing one or more medical conditions associated with an anatomical target region are obtained with the training interface 702. The text data may correspond to the descriptive text of the medical condition 304 described in FIG. 3.

In step 804, the training computation unit 704 generates, using one or more physiological models associated with the anatomical target region, synthesized measurement data of the at least one physiological parameter based on the text data. The measurement data may correspond to the physical signals 302 described in connection with FIG. 3.

In optional step 806, the training computation unit 704 may generate further synthesized measurement data of the physiological parameters based on the synthesized measurement data and one or more functional models associated with one or more medical devices suitable for obtaining the measurement data of the physiological parameters. The further synthesized measurement data may include characteristics of the medical devices, such as noise introduced in the measurement data when acquiring the measurement data with the medical devices. The text data may further describe simulated characteristics of the one or more functional models associated with one or more medical devices, i.e. that the associated measurement data is noise prone.

In step 808, the training computation unit 704 trains the foundation model based on the text data and the synthesized measurement data and/or optionally the further synthesized measurement data.

Optionally, training of the foundation model comprises determining text embeddings associated with the text data by use of a first encoder, see step 810. The first encoder may correspond to the text encoder 408. Furthermore, training of the foundation model may optionally comprise determining measurement embeddings associated with the synthesized measurement data and/or further synthesized measurement data by use of a second encoder, see step 812. The second encoder may correspond to the sequence encoder 410. Parameter values of the foundation model may be updated based on association between the text embeddings and the measurement embeddings, for example based on a contrastive loss between the text embeddings and the measurement embeddings.

Also optionally, training of the foundation model comprises generating further text data by a decoder based on the text embeddings and the measurement embeddings, see step 814. The decoder may correspond to the decoder 502 described in connection with FIG. 5. Parameter values of the foundation model may be updated in addition based on a comparison between the text data and the further text data, for example based on a captioning loss between the text data and to the further text data. Step 816.

Optionally, the training computation unit performs in step 818 a fine-tuning of the foundation model based on manually created text data describing one or more medical conditions associated with the anatomical target region of a patient and clinical measurement data of the at least one physiological parameter associated with the anatomical target region of the patient.

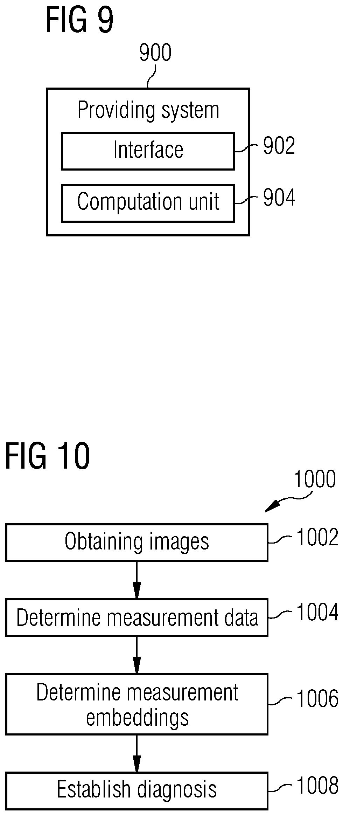

FIG. 9 schematically shows a providing system 900 for establishing a diagnosis of a disease associated with a heart valve. The providing system 900 may be configured to implement the above-described techniques for valve disease assessment.

The providing system 900 comprises an interface 902 configured to obtain a series of medical images depicting the heart valve, and a computation unit 904. For example, the interface 902 may comprise a data communication interface, e.g. a proprietary interface or a standardized interface such as a local area network (LAN) interface, configured to communicate with a medical imaging device such as an ultrasound imaging device or a magnetic resonance imaging (MRI) device. The computation unit 904 may comprise, for example, one or more processing circuits such as a computer. Such circuits and other electrical devices may be combined with each other and/or separated in any manner based on the particular type of electrical implementation that is desired.

For example, the computation unit 904 may be configured to execute the method steps 1002 to 1008 of method 1000 illustrated in FIG. 10.

In step 1002, the computation unit 904 obtains a series of medical images. For example, the series of medical images shows an interior of a beating human heart or at least a part of the heart such as a heart valve. The series of medical images may be a video with a frame rate in a range from 5 to 60 frames per second, for example.

In step 1004, based on the series of medical images, the computation unit 904 determines measurement data of at least one physiological parameter associated with the heart, in particular associated with the heart valve. The physiological parameter may be related to a blood velocity, a left ventricular outflow tract diameter, a transvalvular pressure gradient, or a left ventricular volume. The blood velocity may be a blood velocity at the valve, such as a blood velocity at an aortic valve annulus. The measurement data may be a series of values over time, such as velocity values of one or more heartbeats.

In step 1006, the computation unit 904 determines measurement embeddings associated with the measurement data. The computation unit 904 may utilize an encoder, for example the sequence encoder 406 discussed in connection with FIG. 4, for determining the measurement embeddings.

Based on the determined measurement embeddings, the computation unit 904 establishes in step 1008 the diagnosis of the disease, for example by use of a machine-learning model. The machine-learning model may be integrated in the providing system.

In summary, the proposed training system provides the capability to extract detailed and advanced information from the input signal. The system is trained to learn the association between signals and physiological models - both in terms of physics laws as well as presence of noise and artifacts. The concept of producing training samples programmatically from physiological models also allows to highlight the effect of variations in model parameters and changes in the signals. This can make the trained system sensitive to potentially subtle signal features which can be explained by specific model parameters, thus enabling signal interpretation grounded in physiological principles.

The providing system produces detailed text descriptions of a signal given as input, including references to the underlying physiological model, the model parameter configuration and/or their meaning. In the case of detection and grading of valvular defects, the system produces not only detailed physiologically-based interpretation of the input signal, but can also assess consistency between the input signals, notifying the user in case of inconsistency.

The following point is also part of the disclosure:

A training system for performing a training of a foundation model for processing measurement data of at least one physiological parameter associated with an anatomical target region, comprising:

- a training interface configured for obtaining text data describing one or more medical conditions associated with the anatomical target region;

- a training computation unit configured for

  generating, using one or more physiological models associated with the anatomical target region, synthesized measurement data of the at least one physiological parameter based on the text data, and
  training the foundation model based on the text data and the synthesized measurement data.

List of Reference

| | |
|---|---|
| 100 | neural network |
| 120-132 | node |
| 140-142 | edge |
| 110-113 | layer |

| 200 | convolutional neural network |
| 210 | input layer |
| 211 | convolutional layer |
| 212 | pooling layer |
| 213 | fully connected layer |
| 214 | output layer |
| 220-224 | node |
| 302, 304, 306 | box |
| 308, 310 | physiological signal |
| 312 | box |
| 314 | noise parameters |
| 316 | box |
| 318, 320 | pair |
| 402 | signal input |
| 404 | descriptive text input |
| 406 | sequence encoder |
| 408 | text encoder |
| 410 | signal embedding |
| 412 | text embedding |
| 414 | contrastive loss |
| 502 | decoder |
| 504 | captioning loss |
| 602, 604 | echocardiography image |
| 700 | training system |
| 702 | training interface |
| 704 | training computation unit |
| 800 | method |
| 802-818 | method step |
| 900 | providing system |
| 902 | interface |
| 904 | computation unit |
| 1000 | method |
| 1002-1008 | method step |

**Claims**

1. A computer-implemented method for performing a training of a foundation model for processing measurement data of at least one physiological parameter associated with an anatomical target region, the method (800) comprising:

- obtaining (802) text data (304, 404) describing one or more medical conditions associated with the anatomical target region;
- generating (804), using one or more physiological models associated with the anatomical target region, synthesized measurement data (308, 310, 402) of the at least one physiological parameter (302) based on the text data (304, 404);
- training (808) the foundation model based on the text data (304, 404) and the synthesized measurement data (308, 310).

2. The computer-implemented method of claim 1, further comprising:

- generating (806) further synthesized measurement data of the at least one physiological parameter based on the synthesized measurement data and one or more functional models associated with one or more medical devices suitable for obtaining the measurement data of the at least one physiological parameter;
wherein the text data (304, 404) is further describing simulated characteristics of the one or more functional models associated with one or more medical devices,
wherein the foundation model is trained based on the text data (304, 404) and the further synthesized measurement data.

3. The computer-implemented method of claim 1, wherein the foundation model comprises a first encoder (408) and a second encoder (406), and said training of the foundation model comprises:

- determining (810), using the first encoder (408), one or more text embeddings (412) associated with the text data (304, 404);
- determining (812), using the second encoder (406), one or more measurement embeddings (410) associated with the synthesized measurement data (308, 310, 402);
- updating parameter values of the foundation model based on an association between the one or more text embeddings (412) and the one or more measurement embeddings (410).

4. The computer-implemented method of claim 2, wherein the foundation model comprises a first encoder (408) and a second encoder (406), and said training of the foundation model comprises:

- determining (810), using the first encoder (408), one or more text embeddings (412) associated with the text data (304, 404);
- determining (812), using the second encoder (406), one or more measurement embeddings (410) associated with the further synthesized measurement data;
- updating parameter values of the foundation model based on an association between the one or more text embeddings (412) and the one or more measurement embeddings (410).

5. The computer-implemented method of claim 3 or 4, wherein the synthesized measurement data (308, 310, 402) or the further synthesized measurement data comprises measurement data associated with multiple physiological parameters, and the measurement data associated with each of the multiple physiological parameters are fed to a separate branch of the second encoder.

6. The computer-implemented method of any one of claims 3 to 5, wherein the parameter values of the foundation model are updated based on a contrastive loss (414) between the one or more text embeddings (412) and the one or more measurement embeddings (410).

7. The computer-implemented method of claim 6, wherein the foundation model further comprises a decoder (502), the method (100) further comprising:

- generating (814), using the decoder (502), further text data based on the one or more text embeddings (412) and the one or more measurement embeddings (410);
- wherein the parameter values of the foundation model are updated further based on a comparison (504) between the text data (304, 404) and the further text data.

8. The computer-implemented method of any one of the preceding claims, further comprising:

- fine-tuning (818) the foundation model based on manually created text data describing one or more medical conditions associated with the anatomical target region of a patient and clinical measurement data of the at least one physiological parameter associated with the anatomical target region of the patient.

9. The computer-implemented method of any one of the preceding claims,

wherein the anatomical target region comprises at least one of an aortic valve, a mitral valve, a pulmonary valve, and a tricuspid valve;
wherein the at least one physiological parameter comprises at least one of a blood velocity, a left ventricular outflow tract diameter, a transvalvular pressure gradient, and a left ventricular volume;
wherein the medical conditions comprise a presence of at least one of a stenosis, a regurgitation, a prolapse, and an atresia.

10. A computer-implemented method for establishing a diagnosis of a disease associated with a heart valve, the method (1000) comprising:

- obtaining (1002) a series of medical images depicting the heart valve;
- determining (1004), based on the series of medical images, measurement data of at least one physiological parameter associated with the heart valve;
- determining (1006), using the second encoder (406) of the foundation model according to any one of claims 3 to 9, measurement embeddings associated with the measurement data;
- establishing (1008), using a machine-learning model (502), the diagnosis of the disease based on the measurement embeddings.

11. The computer-implemented method of claim 10, wherein establishing (1008) the diagnosis of the disease is based on text embeddings associated with the measurement embeddings.

12. The computer-implemented method of claim 10 or claim 11, wherein establishing (1008) the diagnosis of the disease is based on further text data generated based on the one or more text embeddings and the one or more measurement embeddings.

13. A providing system for establishing a diagnosis of a disease associated with a heart valve, comprising:

- an interface (902) configured for obtaining (1002) a series of medical images depicting the heart valve;
- a computation unit (904) configured for

determining (1004), based on the series of medical images, measurement data of at least one physiological parameter associated with the heart valve;
determining (1006), using the second encoder (406) of the foundation model according to any one of claims 3 to 9, measurement embeddings associated with the measurement data; and
establishing (1008), using a machine-learning model (502), the diagnosis of the disease based on the measurement embeddings.

14. A computer program comprising instructions which, when the program is executed by a system (700, 900), cause the system (700, 900) to carry out the method (800, 1000) of one of the claims 1 to 12.

15. A computer-readable medium comprising instructions which, when executed by a system (700, 900), cause the system (700, 900) to carry out the method (800, 1000) of one of the claims 1 to 12.

FIG 1

FIG 2

# FIG 3

## FIG 4

402
1-D signal

406
Sequence encoder

Signal embedding
410

414
Contrastive loss

404
Descriptive text

408
Text encoder

412
Text embedding

## FIG 5

402
1-D signal

406
Sequence encoder

Signal embedding
410

414
Contrastive loss

404
Descriptive text

408
Text encoder

412
Text embedding

502
Decoder

504
Captioning loss

FIG 6

Early Systok
LV Outflow    602

Annular
Diameter

Velocity-PW

RV   LV    LV contour    604

FIG 7

700

Training system

Training interface    702

Training computation unit    704

# FIG 8

800

| Obtaining text data | 802 |

↓

| Generating synthesized measurement data | 804 |

↓

| Generating further synthesized measurement data | 806 |

↓

Training foundation model — 808

| Determine text embeddings | 810 |

| Determine measurement embeddings | 812 |

| Generate further text data | 814 |

| Update foundation model parameters | 816 |

↓

| Fine-tuning foundation model | 818 |

FIG 9

900

Providing system

| Interface | — 902 |

| Computation unit | — 904 |

FIG 10

1000

| Obtaining images | — 1002 |

| Determine measurement data | — 1004 |

| Determine measurement embeddings | — 1006 |

| Establish diagnosis | — 1008 |

Europäisches Patentamt
European Patent Office
Office européen des brevets

## EUROPEAN SEARCH REPORT

**Application Number**

EP 25 16 6712

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHRISTENSEN MATTHEW ET AL: "Vision-language foundation model for echocardiogram interpretation", NATURE MEDICINE, [Online] vol. 30, no. 5, 30 April 2024 (2024-04-30), pages 1481-1488, XP093314483, New York ISSN: 1078-8956, DOI: 10.1038/s41591-024-02959-y Retrieved from the Internet: URL:https://escholarship.org/content/qt3918n7bf/qt3918n7bf.pdf> [retrieved on 2025-09-12] | 1-9, 13-15 | INV.<br>G16H30/40<br>G16H50/20<br>G16H50/30<br>G16H50/70 |
| A | * Page 1481, Abstract *<br>* figures 1, 4 * | 10-12 | |
| X | AHMADI N. ET AL: "Transformer-Based Spatio-Temporal Analysis for Classification of Aortic Stenosis Severity From Echocardiography Cine Series", IEEE TRANSACTIONS ON MEDICAL IMAGING, [Disk] vol. 43, no. 1, 1 January 2024 (2024-01-01), pages 366-376, XP093221512, USA ISSN: 0278-0062, DOI: 10.1109/TMI.2023.3305384 * Pages 366-368, Section "I. Introduction" * * Pages 369-370, Section "C. Temporal Encoder" * * Pages 370-371, Section "E. Classification Branch" * * Page 375, Section "IV. Conclusion" * * figures 1, 2, 4 * | 1-15 | |

| | TECHNICAL FIELDS SEARCHED (IPC) |
|---|---|
| | G16H |

The present search report has been drawn up for all claims

| Place of search | Date of completion of the search | Examiner |
|---|---|---|
| Munich | 12 September 2025 | Bonnet, Clara |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CARO, C. G.** ; **T. J. PEDLEY** ; **R. C. SCHROTER** ; **W. A. SEED** ; **K. H. PARKER**. The Mechanics of the Circulation. Cambridge University Press, 2011
- **ZOGHBI, WILLIAM A.** ; **DAVID ADAMS** ; **ROBERT O. BONOW** ; **MAURICE ENRIQUEZ-SARANO** ; **ELYSE FOSTER** ; **PAUL A. GRAYBURN** ; **REBEC-CA T. HAHN et al.** Recommendations for Noninvasive Evaluation of Native Valvular Regurgitation. *Journal of the American Society of Echocardiography*, April 2017, vol. 30 (4), 303-71

- **LE, HUONG T.** ; **NICHOLAS HANGIANDREOU** ; **ROBERT TIMMERMAN** ; **MARK J. RICE** ; **W. BRIT SMITH** ; **LORI DEITTE** ; **GREGORY M. JANELLE**. Imaging Artifacts in Echocardiography. *Anesthesia & Analgesia*, March 2016, vol. 122 (3), 633-46
- **YU, HAN** ; **PEIKUN GUO** ; **AKANE SANO**. ECG Semantic Integrator (ESI): A Foundation ECG Model Pretrained with LLM-Enhanced Cardiological Text.. *arXiv*, 26 May 2024
- **YU, HAN** ; **PEIKUN GUO** ; **AKANE SANO**. ECG Semantic Integrator (ESI): A Foundation ECG Model Pretrained with LLM-Enhanced Cardiological Text. *arXiv*, 26 May 2024